(19) Europäisches Patentamt
European Patent Office
Office européen des brevets

(11) Publication number: **0 065 301**
**A1**

(12) **EUROPEAN PATENT APPLICATION**

(21) Application number: **82104291.8**

(22) Date of filing: **17.05.82**

(51) Int. Cl.³: **C 07 C 103/52**, A 61 K 37/02

(30) Priority: **18.05.81 US 264880**
**29.03.82 US 362082**

(43) Date of publication of application: **24.11.82**
**Bulletin 82/47**

(84) Designated Contracting States: **AT BE CH DE FR GB IT LI LU NL SE**

(71) Applicant: **MERCK & CO. INC., 126, East Lincoln Avenue P.O. Box 2000, Rahway, New Jersey 07065 (US)**

(72) Inventor: **Patchett, Arthur A., 1090 Minisink Way, Westfield New Jersey 07090 (US)**
Inventor: **Wu, Mu Tsu, 36 Lance Drive, Clark New Jersey 07066 (US)**

(74) Representative: **Abitz, Walter, Dr.-Ing. et al, Abitz, Morf, Gritschneder, Freiherr von Wittgenstein Postfach 86 01 09, D-8000 München 86 (DE)**

(54) **Isoquinoline carboxylic acid derivates, process for preparing and pharmaceutical composition containing the same.**

(57) The invention relates to isoquinoline carboxylic acid derivatives and related compounds of the formula:

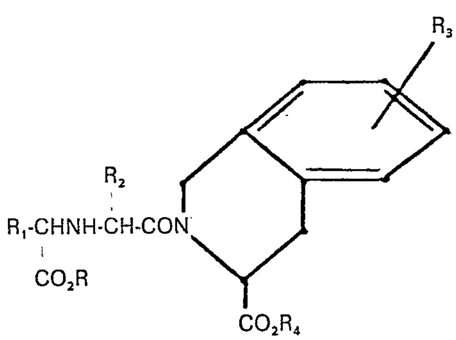

wherein:

R and $R_4$ are hydrogen, loweralkyl, aralkyl;

$R_1$ is alkyl of from 1 to 10 carbon atoms; substituted alkyl of from 1 to six carbon atoms; aralkyl and heteroaralkyl optionally substituted in the aryl and heteroaryl groups;

$R_2$ is hydrogen, alkyl of from 1 to six carbon atoms optionally substituted;

$R_3$ is hydrogen, halo, or alkoxy; and,

the pharmaceutically acceptable salts thereof;

which are useful as angiotensin converting enzyme inhibitors and as antihypertensives; process for preparing and pharmaceutical composition containing the same.

EP 0 065 301 A1

TITLE OF THE INVENTION

ISOQUINOLINE CARBOXYLIC ACID DERIVATIVES, PROCESS FOR PREPARING AND PHARMACEUTICAL COMPOSITION CONTAINING THE SAME

BACKGROUND OF INVENTION

This invention relates to isoquinoline carboxylic acid derivatives and related compounds which are useful as converting enzyme inhibitors and as antihypertensives. The compounds of this invention are represented by the following formula:

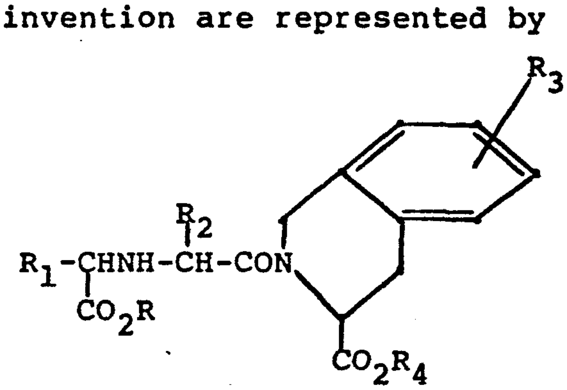

(I)

wherein

R and $R_4$ are independently hydrogen, loweralkyl, aralkyl;

$R_1$ is alkyl of from 1 to 10 carbon atoms which include branched, cyclic and unsaturated alkyl groups;

substituted alkyl of from 1 to six carbon atoms and the substituent is amino, arylthio, aryloxy, hydroxy, arylamino and acylamino;

aralkyl and heteroaralkyl optionally substituted in the aryl and heteroaryl groups by halo, loweralkyl, hydroxy, alkoxy, amino, and aminoalkyl groups and optionally substituted in the $C_1$-$C_6$ alkyl groups by amino, acylamino or hydroxy groups;

$R_2$ is hydrogen, alkyl of from 1 to six carbon atoms optionally substituted by amino, alkylamino, arylamino, acylamino, heteroaryl, or aryl groups;

$R_3$ is hydrogen, halo, or alkoxy; and,

the pharmaceutically acceptable salts thereof.

The loweralkyl groups, except where noted otherwise, represented by any of the variables include straight and branched chain hydrocarbon radicals of from one to six carbon atoms such as, for example, methyl, ethyl, propyl, isopropyl, butyl, isobutyl, t-butyl, pentyl, isopentyl, hexyl or vinyl, allyl, butenyl, and the like. The aralkyl groups represented by any of the above variables have from one to four carbon atoms in the alkyl portion thereof and include, for example, benzyl, phenethyl, and the

like. Halo means chloro, bromo, iodo or fluoro. Aryl where it appears in any of the radicals, except where noted, represents phenyl, or naphthyl and heteroaryl includes, for example, indolyl, thienyl, quinolinyl, isoquinolinyl, imidazolyl, furyl, pyridyl and benzothienyl. Heteroaralkyl indicates that these same residues are attached to 1-4 carbon atoms and include, for example, indolylethyl, 1-isoquinolinylmethyl, and the like. Acylamino refers to loweralkanoylamino and aroylamino groups.

Preferred are those compounds of Formula I wherein:

R and $R_4$ are independently hydrogen, loweralkyl, and aralkyl;

$R_1$ is alkyl of from 1 to 6 carbon atoms optionally substituted by amino, hydroxyl, acylamino, aryloxy, arylthio, aralkyl and heteroaralkyl wherein the aryl and heteroaryl groups may optionally be substituted by halo, loweralkyl, hydroxy, alkoxy, amino and aminoalkyl groups;

$R_2$ is alkyl of from 1 to 6 carbon atoms optionally substituted by amino, heteroaryl, or aminoloweralkylthio groups; and,

$R_3$ is hydrogen.

Still more preferred are those compounds of Formula I wherein R is hydrogen, loweralkyl; and $R_1$, $R_2$ and $R_3$ are as defined in the preferred compounds above.

Most preferred are compounds of Formula I wherein:

R and $R_4$ are independently hydrogen or loweralkyl;

$R_1$ is aralkyl or heteroaralkyl optionally substituted by halo or aminoalkyl groups;

$R_2$ is alkyl of from 1 to 6 carbon atoms optionally substituted by amino or aminoloweralkythio groups; and,

$R_3$ is hydrogen.

The preferred, more preferred and most preferred compounds also include the pharmaceutically acceptable salts thereof.

The compounds of this invention can be synthesized by the methods described hereinbelow. Unless otherwise indicated, starting materials required for these syntheses are known in the literature or can be made by known methods using known starting materials.

Interfering functionality in the starting materials such as, for example, hydroxy, carboxy or amino groups, can be masked by protecting groups which can then be subsequently removed to afford desired end products. Appropriate protecting groups are well known to those skilled in the art of peptide chemistry, some of which are illustrated in the processes described herein.

In the following description of the invention processes, R, $R_1$, $R_2$, $R_3$ and $R_4$ are as defined above, unless otherwise indicated.

REACTION SCHEME

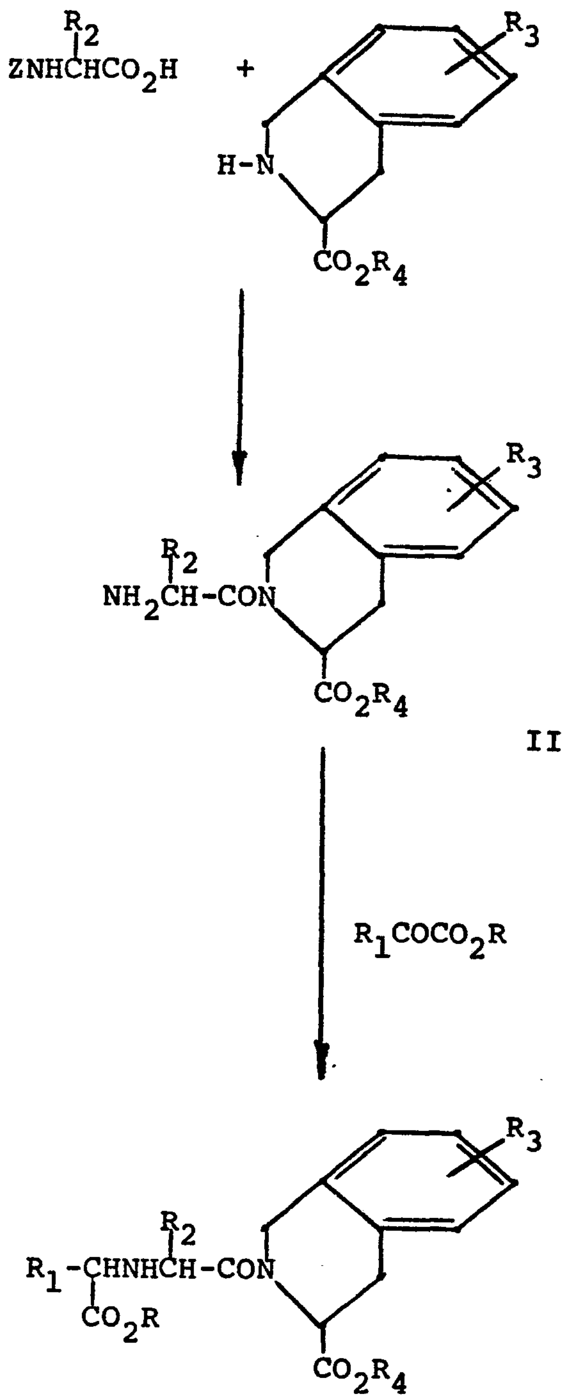


II

(I)

With reference to the foregoing Reaction Scheme, dipeptide analog II is synthesized by coupling an α-blocked amino acid (for example, Z=carbobenzyloxy or t-butoxycarbonyl) with an isoquinoline ester using appropriate reagents such as, for example, dicyclohexyl carbodiimide or diphenylphosphoryl azide. The Z protecting group is removed using standard methods and R can then be taken off of the isoquinoline carboxylate by saponification. The α-keto acids or esters containing the $R_1$ substituent are then reductively added to compound II. This can be conveniently accomplished using sodium cyanoborohydride in a mixed aqueous solvent at approximately neutral pH or by hydrogenation in an inert solvent in the presence of Pd/C or Raney nickel. Removal of protecting groups, if present, affords the desired products of Formula I.

The carbon atoms to which $R_1$ and $R_2$ are attached and the asymmetric carbon in the isoquinoline ring of Formula I compounds are all preferably of the S absolute configuration. Other diastereomers in which an R configuration is present also exhibit activity and are, therefore, also included within the scope of this invention. Preferred Formula I diastereomers are isolated either by chromatography or fractional crystallization.

The α-ketocarboxylate derivatives used in the reductive alkylation can include such compounds as, for example, ethyl 2-oxo-4-phenylbutyrate, ethyl 4-*p*-chlorophenyl-2-oxobutyrate, ethyl 4-(4-imidazolyl)-2-oxobutyrate, ethyl 2-oxo-4-(2-thienyl)-butyrate, ethyl 2-oxo-4-(2-napthyl)-butyrate, ethyl 4-p-hydroxy-phenyl-2-oxobutyrate, ethyl phenoxypyruvate, ethyl 2-oxo-5-phenyl-pentanoate, ethyl 4-p-methoxyphenyl-2-oxobutyrate, ethyl 5-methyl-2-oxohexanoate, benzyl 2-oxo-6-phthalimidohexanoate, and the like, and their respective free acids.

The compounds of this invention form salts with various inorganic and organic acids and bases which are also within the scope of the invention. Such salts include ammonium salts, alkali metal salts like sodium and potassium salts, alkaline earth metal salts like the calcium and magnesium salts, salts with organic bases e.g., dicyclohexylamine salts, N-methyl-D-glucamine, salts with amino acids like arginine, lysine, and the like. Also, salts with organic and inorganic acids may be prepared, e.g., HCl, HBr, $H_2SO_4$, $H_3PO_4$, methanesulfonic, toluenesulfonic, maleic, fumaric, camphorsulfonic. The non-toxic physiologically acceptable salts are preferred, although other salts are also useful, e.g., in isolating or purifying the product.

The salts may be formed by conventional means as by reacting the free acid or free base forms of the product with one or more equivalents of the appropriate base or acid in a solvent or medium in which the salt is insoluble, or in a solvent such as water which is then removed <u>in vacuo</u> or by freeze-drying or by exchanging the cations of an existing salt for another cation on a suitable ion exchange resin.

The compounds of this invention inhibit angiotensin converting enzyme and thus block conversion of the decapeptide angiotensin I to angiotensin II. Angiotensin II is a potent pressor substance. Thus blood-pressure lowering can result from inhibition of its biosynthesis especially in animals and humans whose hypertension is angiotensin II related. Furthermore, converting enzyme degrades the vasodepressor substance, bradykinin. Therefore, inhibitors of angiotensin converting enzyme may lower blood-pressure also by potentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, D. W. Cushman et al., Biochemistry 16, 5484 (1977).

The evaluation of converting enzyme inhibitors is guided by *in vitro* enzyme inhibition assays. For example, a useful method is that of Y. Piquilloud, A. Reinharz and M. Roth, Biochem. Biophys. Acta, 206, 136 (1970) in which the hydrolysis of carbobenzyloxyphenylalanylhistidinyl-leucine is measured. *In vivo* evaluations may be made, for example, in normotensive rats challenged with angiotensin I by the technique of J. R. Weeks and J. A. Jones, Proc. Soc. Exp. Biol. Med., 104, 646 (1960) or in a high renin rat model such as that of S. Koletsky et al., Proc. Soc. Exp. Biol. Med. 125, 96 (1967).

Thus, the compounds of this invention are useful as antihypertensives in treating hypertensive mammals, including humans, and they can be utilized to achieve the reduction of blood pressure by formulating in compositions such as tablets, capsules or elixirs for oral administration or in sterile solutions or suspensions for parenteral administration. The compounds of this invention can be administered to patients in need of such treatment in a dosage range of 1.0 to 200 mg per patient generally given several times, thus giving a total daily dose of from 1.0 to 800 mg per day. The dose will vary depending on severity of disease, weight of patient and other factors which a person skilled in the art will recognize.

Also, the compounds of this invention may be given in combination with diuretics or other antihypertensives. Typically, these are combinations whose individual per day dosages range from one-fifth of the minimally recommended clinical dosages to the maximum recommended levels for the entities when they are given singly. To illustrate these combinations, one of the antihypertensives of this invention, effective clinically in the range 15-200 milligrams per day, can be effectively combined at levels ranging from 3-200 milligrams per day with the following antihypertensives and diuretics in dose ranges per day as indicated:
hydrochlorothiazide (15-200 mg), timolol (5-60 mg), methyldopa (65-2000 mg), the pivalyloxyethyl ester of methyldopa (30-1000 mg), indacrinone and variable

ratios of its enantiomers (25-125 mg), and (+)-4-{3-{[2-(1-hydroxycyclohexyl)ethyl]-4-oxo-2-thiazolidinyl} propyl}-benzoic acid (10-100 mg).

In addition, the triple drug combinations of hydrochlorothiazide (10-100 mg) plus amiloride (5-20 mg) plus converting enzyme inhibitor of this invention (3-200 mg) or hydrochlorothiazide (10-100 mg) plus timolol (5-60 mg) plus the converting enzyme inhibitor of this invention (3-200 mg) are effective combinations to control blood pressure in hypertensive patients.

The above dose ranges will be adjusted on a unit basis as necessary to permit divided daily dosage. Also, the dose will vary depending on the severity of the disease, weight of patient and other factors which a person skilled in the art will recognize.

Typically the combinations shown above are formulated into pharmaceutical compositions as discussed below.

About 1.0 to 100 mg of a compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid, and the like; a lubricant such as magnesium stearate; a sweetening agent such as sucrose, lactose or saccharin; a flavoring agent such as peppermint, oil of wintergreen or cherry. When the dosage unit form is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc. or a synthetic fatty vehicle like ethyl oleate or the like. Buffers, preservatives, antioxidants and the like can be incorporated as required.

The following examples are illustrative of the invention and constitute especially preferred

embodiments. The preferred diastereomers of these examples are isolated by conventional column chromatography or fractional crystallization. Unless otherwise indicated, all temperatures are in degrees Celsius.

EXAMPLE 1

2-[$N^{\alpha}$-(1-Carboxy-3-phenylpropyl)-L-lysyl]-L-1,2,3,4-tetrahydroisoquinoline-3-carboxylic acid

α-t-BOC-ε-CBZ-L-lysine and ethyl 1,2,3,4-tetrahydroisoquinoline-3(S)-carboxylate are condensed in the presence of N,N'-dicyclohexylcarbodiimide. The ethyl ester is hydrolyzed by stirring in ethanolic sodium hydroxide (1.3 eq.) at room temperature for 22 hours. The resulting sodium salt is stirred in 4N HCl in EtoAc at room temperature for 1 hour to remove the t-BOC group. The hydrochloride salt (1.48 mmol) and 2-oxo-4-phenylbutyric acid (1.32 g, 7.40 mmol) are dissolved in 50% ethanol and neutralized to pH 7 with dilute sodium hydroxide. A solution of sodium cyanoborohydride (279 mg, 4.44 mmol, in 5 ml of ethanol) is added by syringe pump at the rate of 1.5 ml/hr. After several days stirring at room temperature, the product is absorbed on strong acid ion exchange resin (Dowex 50 ($H^+$), 50-100 mesh). The column is rinsed to pH 6 with water, then eluted with 2% pyridine in water. Product rich cuts are evaporated to a white solid (595 mg., 67%). The solid is dissolved in methanol and purified by LH-20 gel filtration chromatography. This procedure reveals the presence of a substantial

amount (~50%) of the corresponding diketopiperazine compound as a contaminant. The pure product obtained above is stirred in 30% HBr in glacial acetic acid for 15 minutes at room temperature to remove the CBZ protecting group. The free amine is obtained after treatment of the HBr salt with Dowex 50 ($H^+$). The final product, 2-[$N^{\alpha}$-(1-carboxy-3-phenylpropyl)-L-lysyl]-L-1,2,3,4-tetrahydroisoquinoline-3-carboxylic acid, is freeze dried from water containing several drops of methanol as a white fluffy solid (135 mg. 0.29 mmol, 18% overall yield). The nmr spectrum confirmed this structure. The mass spectrum shows a molecular ion at m/e 683 for the trisilylated species; m/e 668 for loss of methyl from the trisilyl; m/e 566 for loss of - $CO_2$TMS from the trisilyl; and m/e 407 for the common fragment,

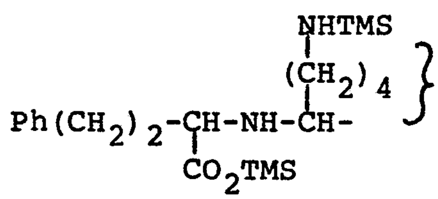

EXAMPLE 2

2-[N-(1-Carbethoxy-3-phenylpropyl)-L-alanyl]-L-1,2,3,4-tetrahydroisoquinoline-3-carboxylic acid

CBZ-L-alanine and ethyl 1,2,3,4-tetrahydroisoquinoline-3(S)-carboxylate are condensed in the presence of N,N'-dicyclohexylcarbodiimide. Hydrolysis of the ethyl ester is accomplished by stirring in ethanolic sodium hydroxide (1.5 eq) at room temperature overnight. The resulting sodium

salt is stirred in 30% HBr in glacial acetic acid for 1 hour at room temperature to remove the CBZ protecting group. The free amine is obtained from the HBr salt by treatment with Dowex 50 ($H^+$). 2-(L-Alanyl-L-1,2,3,4-tetrahydro-isoquinoline-3-carboxylic acid (234 mg., 0.942 mmol) and ethyl 2-oxo-4-phenylbutyrate (971 mg., 4.71 mmol) are dissolved in 80 ml of absolute ethanol. To this solution is added 1.6 g of 4A powdered molecular sieves. A solution of sodium cyanoborohydride (177 mg., 2.83 mmol, in 5 ml of EtOH) is added to this suspension by syringe pump at the rate of 1 ml/hr. After stirring at room temperature for several days, the reaction mixture is filtered to remove molecular sieves and stripped to a yellow viscous oil. The oil is stirred with Dowex 50 ($H^+$) in a mixture of water and ethanol for 1 hour. This mixture is then added to a Dowex 50 ($H^+$) (50-100 mesh) column packed with 50% EtOH. The column is washed to pH 5 6 with 50% EtOH, then water. The product is then eluted with 2% pyridine in water. Product rich cuts are stripped to a gummy solid (256 mg., 62%). The crude product is purified by LH-20 gel filtration chromatography. The purified product is essentially water insoluble and is freeze dried from dioxane as a thick gum. The nmr spectrum is consistent with structure.

## EXAMPLE 3

2-[N-(1-Carboxy-3-phenylpropyl)-L-alanyl]-L-1,2,3,4-tetrahydroisoquinoline-3-carboxylic acid

Ethyl ester (140 mg., 0.32 mM) in Example 2, was dissolved in 0.5 ml of methanol, 1.0 ml of 1 M NaOH was added and the solution was stirred at room temperature overnight. The solution was acidified with dilute hydrochloric acid and concentrated *in vacuo*. The residue was applied to a 240 X 0.9 cm LH-20 column and eluted with 3 l fractions of methanol. Concentration of fractions 47-58 gave 69 mg (53%) of 2-[N-(1-carboxy-3-phenylpropyl)-L-alanyl]-L-1,2,3,4-tetrahydroisoquinoline-3-carboxylic acid as a white solid after freeze drying; tlc (4-ethyl acetate: 1-acetic acid with Anal. Tech SGF mini plate): Rf = 0.92 and 0.88; 200 MH nmr (deuteromethanol): consistent; mass spectrum (trimethylsilylated): m/e 626.

## EXAMPLE 4

As illustrated in the above example, keto acids and esters listed in Table I below can be reductively condensed using sodium cyanoborohydride with the dipeptides L-alanyl-L-1,2,3,4-tetrahydroisoquinoline-3-carboxylic acid and ε-Cbz-L-lysyl-L-1,2,3,4-tetrahydroisoquinoline-3-carboxylic acid to yield additional products of Formula I as listed in Table II below.

<u>TABLE I</u>

<u>Keto Acids and Esters</u>

<u>$R_1CO-CO_2R$</u>

(a) $(CH_3)_2CH-CH_2COCO_2Et$

(b) 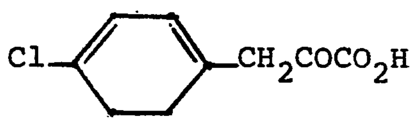

(c) 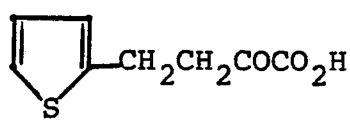

(d) 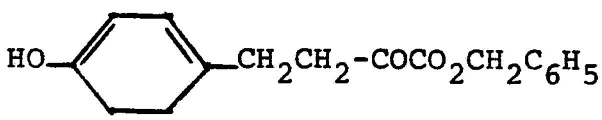

(e) 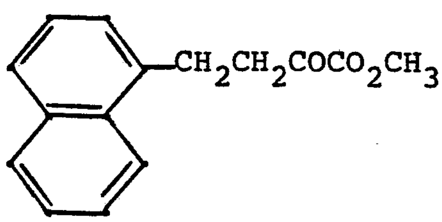

(f) $CH_3S-CH_2CH_2-CO-CO_2H$

(g) 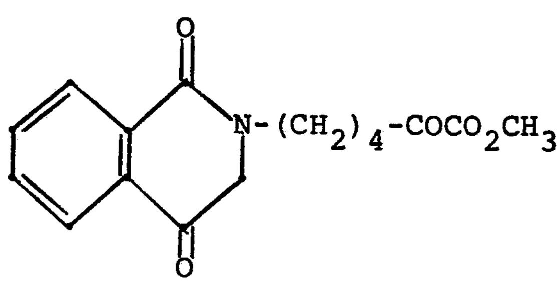

(h)

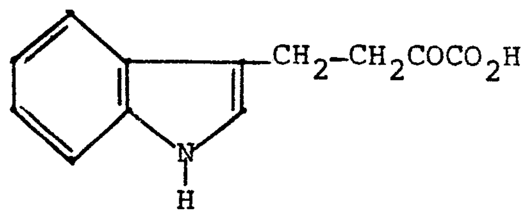

(i)

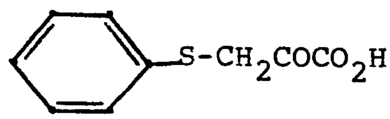

(j)

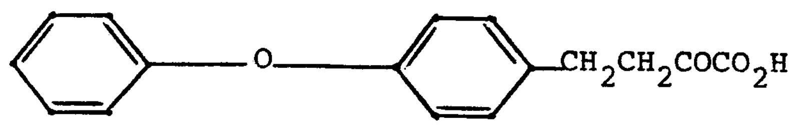

(k)

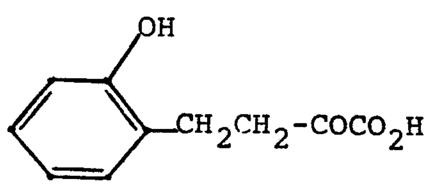

(l)

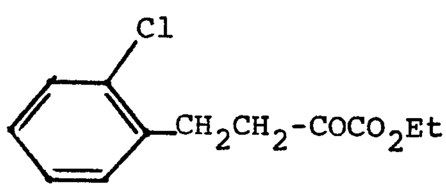

(m)

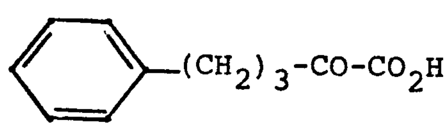

(n) $(CH_3)_2-CH-CH_2CH_2-CO-CO_2H$

(o)

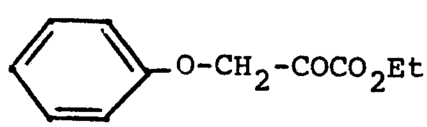

(p)

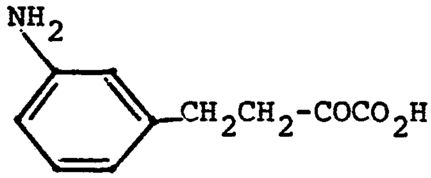

(q)

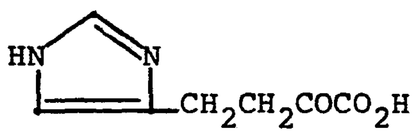

TABLE II

ADDITIONAL PRODUCTS OF FORMULA I

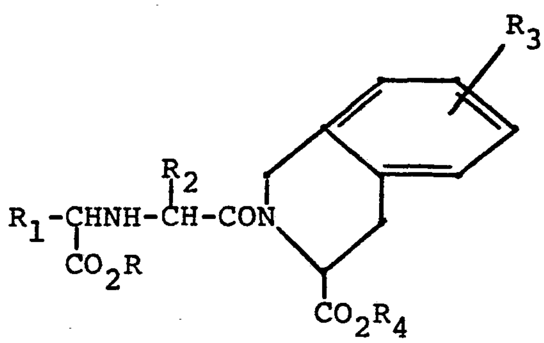

($R_3$ = $R_4$ = H)

| | R | $R_1$ | $R_2$ |
|---|---|---|---|
| (1) | Et | $(CH_3)_2CH-CH_2$ | $CH_3-$ |
| (2) | H | 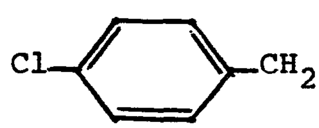 | $NH_2(CH_2)_4-$ |
| (3) | H | 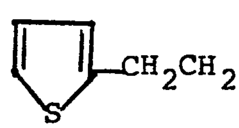 | $CH_3-$ |
| (4) | $C_6H_5-CH_2-$ | 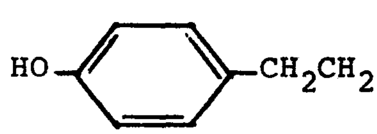 | $NH_2(CH_2)_4-$ |

| | | |
|---|---|---|
| (5) $CH_3$ | 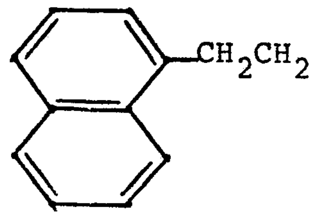 | $CH_3$- |
| ( 6) H | $CH_3S$-$CH_2CH_2$- | $NH_2(CH_2)_4$- |
| ( 7) $CH_3$ | $H_2N(CH_2)_4$- | $CH_3$- |
| ( 8) H | 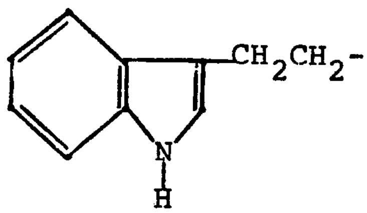 | $CH_3$- |
| ( 9) H | 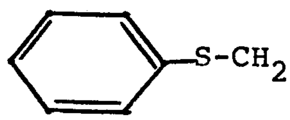 | $NH_2(CH_2)_4$- |
| (10) H | 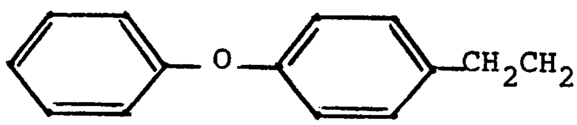 | $CH_3$ |
| (11) H | 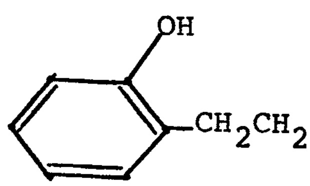 | $NH_2(CH_2)_4$- |

| | | |
|---|---|---|
| (12) Et | 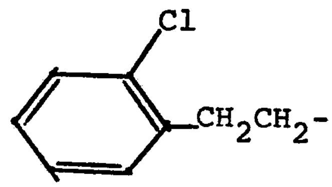 | $CH_3$ |
| (13) H | 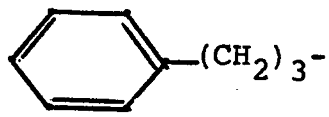 | $NH_2(CH_2)_4$- |
| (14) H | $(CH_3)_2CH-CH_2CH_2$- | $CH_3$ |
| (15) Et | 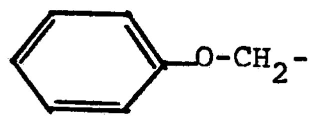 | $CH_3$ |
| (16) H | 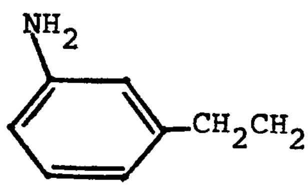 | $CH_3$ |
| (17) H | 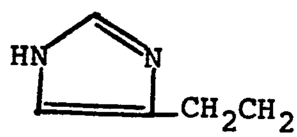 | $NH_2(CH_2)_4$- |

WHAT IS CLAIMED IS:

1. A compound of the formula:

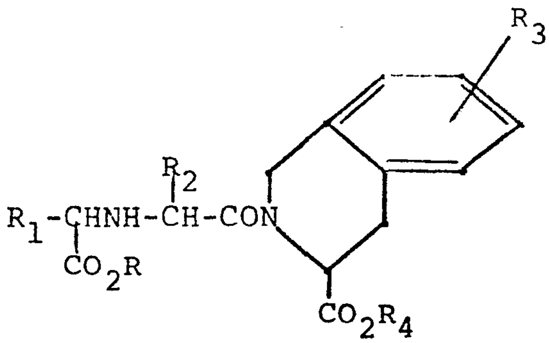

wherein:

R and $R_4$ are independently hydrogen, loweralkyl, aralkyl;

$R_1$ is alkyl of from 1 to 10 carbon atoms which include branched, cyclic and unsaturated alkyl groups;
substituted alkyl of from 1 to six carbon atoms and the substituent is amino, arylthio, aryloxy, hydroxy, arylamino and acylamino;
aralkyl and heteroaralkyl optionally substituted in the aryl and heteroaryl groups by halo, loweralkyl, hydroxy, alkoxy, amino, and aminoalkyl groups and optionally substituted in the $C_1$-$C_6$ alkyl groups by amino, acylamino or hydroxy groups;

$R_2$ is hydrogen, alkyl of from 1 to six carbon atoms optionally substituted by amino, alkylamino, arylamino, acylamino, heteroaryl, or aryl groups;

$R_3$ is hydrogen, halo, or alkoxy; and, the pharmaceutically acceptable salts thereof.

2. A compound of Claim 1 which is a member of the group:

2-[$N^{\alpha}$-(1(S)-carboxy-3-phenylpropyl)-L-lysyl]-L-1,2,3,4-tetrahydroisoquinoline-3-carboxylic acid;

2-[N-(1(S)-carboxy-3-phenylpropyl)-L-alanyl]-L-1,2,3,4-tetrahydroisoquinoline-3-carboxylic acid; and,

2-[N-(1(S)-carboethoxy-3-phenylpropyl)-L-alanyl]-L-1,2,3,4-tetrahydroisoquinoline-3-carboxylic acid.

3. A pharmaceutical composition useful in the treatment of hypertension which comprises a pharmaceutically acceptable carrier and an antihypertensively effective amount of a compound of the formula:

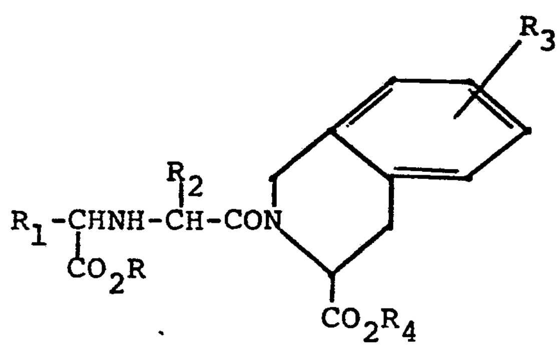

wherein:

R and $R_4$ are independently hydrogen, loweralkyl aralkyl;

$R_1$ is alkyl of from 1 to 10 carbon atoms which include branched, cyclic and unsaturated alkyl groups;

substituted alkyl of from 1 to six carbon atoms and the substituent is amino, arylthio, aryloxy, hydroxy, arylamino and acylamino; aralkyl and heteroaralkyl optionally substituted in the aryl and heteroaryl groups by halo, loweralkyl, hydroxy, alkoxy, amino, and aminoalkyl groups and optionally substituted in the $C_1$-$C_6$ alkyl groups by amino, acylamino or hydroxy groups;

$R_2$ is hydrogen, alkyl of from 1 to six carbon atoms optionally substituted by amino, alkylamino, arylamino, acylamino, heteroaryl, or aryl groups;

$R_3$ is hydrogen, halo or alkoxy; and,

the pharmaceutically acceptable salts thereof.

4. The composition of Claim 3 which includes an antihypertensive and/or diuretic compound selected from the group consisting of hydrochlorothiazide, methyldopa, timolol, the pivalyloxyethyl ester of methyldopa, indacrinone and variable ratios of its enantiomers, (+)-4-{3-{[2-(1-hydroxycyclohexyl)-ethyl]-4-oxo-2-thiazolidinyl}propyl}benzoic acid, as well as admixtures and combinations thereof.

5. A process for preparing a compound of the formula:

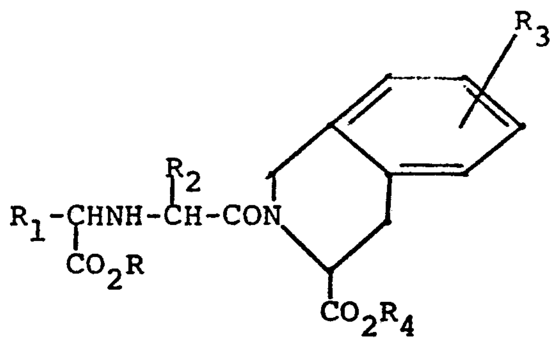

wherein R, $R_1$, $R_2$ $R_3$ and $R_4$ are as defined in Claim 1, which comprises:

reacting an α-keto acid or ester having the formula:

$$R_1-COCO_2-R$$

wherein R and $R_1$ are as defined in Claim 1 and $R_1$ can include suitable protection of reactive groups, with a dipeptide or protected dipeptide analog of the formula:

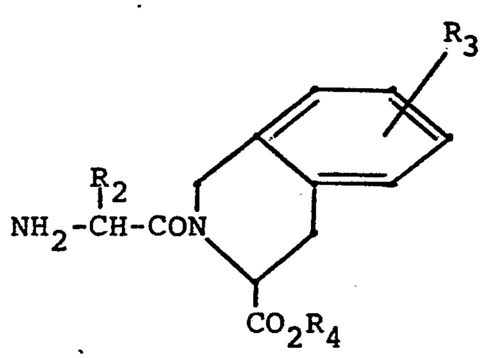

wherein $R_2$, $R_3$ and $R_4$ are as defined in Claim 1 and $R_2$ can include suitable protecting groups, in the presence of a reducing agent, followed by removal of protecting groups, if present, to afford the desired product; and, if further desired, isolating

the biologically more active isomer by chromatography or fractional crystallization and, if still further desired, forming a salt thereof by standard means.

CLAIMS FOR AUSTRIA

1. A process for preparing a compound of the formula:

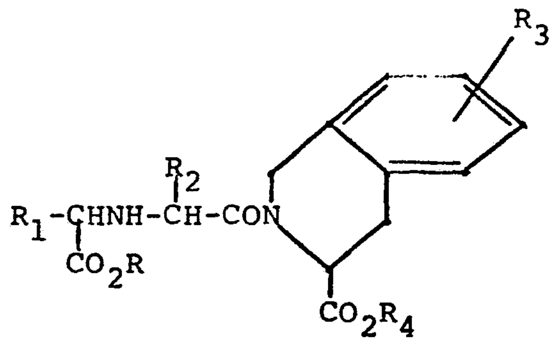

wherein:

R and $R_4$ are independently hydrogen, loweralkyl, aralkyl;

$R_1$ is alkyl of from 1 to 10 carbon atoms which include branched, cyclic and unsaturated alkyl groups;
substituted alkyl of from 1 to six carbon atoms and the substituent is amino, arylthio, aryloxy, hydroxy, arylamino and acylamino;
aralkyl and heteroaralkyl optionally substituted in the aryl and heteroaryl groups by halo, loweralkyl, hydroxy, alkoxy, amino, and aminoalkyl groups and optionally substituted in the $C_1$-$C_6$ alkyl groups by amino, acylamino or hydroxy groups;

$R_2$ is hydrogen, alkyl of from 1 to six carbon atoms optionally substituted by amino, alkylamino, arylamino, acylamino, heteroaryl, or aryl groups;

$R_3$ is hydrogen, halo, or alkoxy; and, the pharmaceutically acceptable salts thereof, which comprises

reacting an α-keto acid or ester having the formula:

$$R_1\text{-}COCO_2\text{-}R$$

wherein R and $R_1$ are defined as above and $R_1$ can include suitable protection of reactive groups, with a dipeptide or protected dipeptide analog of the formula:

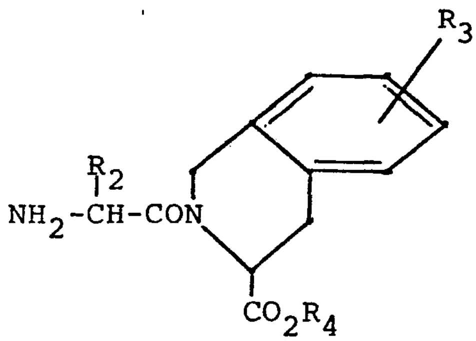

wherein $R_2$, $R_3$ and $R_4$ are defined as above and $R_2$ can include suitable protecting groups, in the presence of a reducing agent, followed by removal of protecting groups, if present, to afford the desired product; and, if further desired, isolating the biologically more active isomer by chromatography or fractional crystallization and, if still further desired, forming a salt thereof by standard means.

2. A process according to claim 1 for preparing a compound which is a member of the group:

2-[$N^{\alpha}$-(1(S)-carboxy-3-phenylpropyl)-L-lysyl]-L-1,2,3,4-tetrahydroisoquinoline-3-carboxylic acid;

2-[N-(1(S)-carboxy-3-phenylpropyl)-L-alanyl]-L-1,2,3,4-tetrahydroisoquinoline-3-carboxylic acid; and,

2-[N-(1(S)-carboethoxy-3-phenylpropyl)-L-alanyl]-L-1,2,3,4-tetrahydroisoquinoline-3-carboxylic acid.

European Patent Office

0065301

# EUROPEAN SEARCH REPORT

Application number

EP 82 10 4291

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| P,X | EP - A - 0 049 605 (WARNER-LAMBERT)<br>* whole patent * | 1-3,5 | C 07 C 103/52<br>A 61 K 37/02 |
| | --- | | |
| P,X | EP - A - 0 049 658 (SCIENCE UNION)<br>* whole patent * | 1-5 | |
| | --- | | |
| P,X | EP - A - 0 046 953 (HOECHST)<br>* whole patent * | 1-5 | |
| | ------- | | |
| | | | TECHNICAL FIELDS SEARCHED (Int. Cl. 3) |
| | | | C 07 C 103/00<br>C 07 D 217/00<br>A 61 K 37/00 |

The present search report has been drawn up for all claims

| Place of search | Date of completion of the search | Examiner |
|---|---|---|
| The Hague | 16.06.1982 | GRAMAGLIA |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82